(19) Europäisches Patentamt
European Patent Office
Office européen des brevets

(11) Veröffentlichungsnummer: **0 398 313**
**A2**

# (12) EUROPÄISCHE PATENTANMELDUNG

(21) Anmeldenummer: **90109285.8**

(22) Anmeldetag: **16.05.90**

(51) Int. Cl.5: **C08B 37/00, A61K 31/715, A61K 35/78**

(30) Priorität: **16.05.89 DE 3915928**
**22.01.90 DE 4001756**

(43) Veröffentlichungstag der Anmeldung:
**22.11.90 Patentblatt 90/47**

(84) Benannte Vertragsstaaten:
**AT BE CH DE DK ES FR GB GR IT LI LU NL SE**

(71) Anmelder: **Ozel, Huseyin Ziya**
**14 Emel Ap. D. 706, Yildiz Posta Cad. 14/706**
**Gayrettepe Istanbul(TR)**

Anmelder: **Hüsnü can Baser, Kemal, Prof. Dr.**
**Arifiye mh. Inhisar sk.**
**38/3 Eskisehir(TR)**

Anmelder: **Carbik, Ismail, Doz. Dr.**
**Franz-Sperr-Weg 6**
**D-8000 München 50(DE)**

Anmelder: **Wagner, Hildebert, Prof. Dr.**
**Nelkenweg 9**
**D-8211 Breitbrunn am Chiemsee(DE)**

(72) Erfinder: **Ozel, Huseyin Ziya**
**14 Emel Ap. D. 706, Yildiz Posta Cad. 14/706**
**Gayrettepe Istanbul(TR)**
Erfinder: **Hüsnü can Baser, Kemal, Prof. Dr.**
**Arifiye mh. Inhisar sk.**
**38/3 Eskisehir(TR)**
Erfinder: **Carbik, Ismail, Doz. Dr.**
**Franz-Sperr-Weg 6**
**D-8000 München 50(DE)**
Erfinder: **Wagner, Hildebert, Prof. Dr.**
**Nelkenweg 9**
**D-8211 Breitbrunn am Chiemsee(DE)**

(74) Vertreter: **Dipl.-Ing. Schwabe, Dr. Dr. Sandmair, Dr. Marx**
**Stuntzstrasse 16**
**D-8000 München 80(DE)**

(54) **Polysaccharide mit immunstimulierender und antiproliferativer Wirkung, Vérfahren zu ihrer Gewinnung und Arzneimittel enthaltend diese Substanzen.**

(57) Die Erfindung betrifft neue Polysaccharide mit immunstimulierender und antiproliferativer Wirkung aus Pflanzen der Gattung Nerium oleander. Eines der isolierten Polysaccharide, besteht aus $\alpha$ (1→4) verknüpften D-Galakturonsäureeinheiten und weist ein Molekulargewicht von 30 000 bis 40 000 D auf. Es wurde weiterhin ein niedrigmolekulares Polysaccharidgemisch mit einem Molekulargewicht von etwa 3 000 bis 12 000 D, das drei Unterfraktionen von Polysacchariden mit unterschiedlichem Molekulargewicht und unterschiedlicher Zusammensetzung enthält, isoliert.

EP 0 398 313 A2

# Polysaccharide mit immunstimulierender und antiproliferativer Wirkung, Verfahren zu ihrer Gewinnung und Arzneimittel enthaltend diese Substanzen

Die Erfindung betrifft Polysaccharide mit immunstimulierender Wirkung, Verfahren zu deren Gewinnung sowie Arzneimittel enthaltend diese Polysaccharide.

Die Immunstimulation als therapeutisches Konzept ist in der Medizin seit langem bekannt. Im allgemeinen versteht man darunter die Verabreichung von Substanzen, die selbst nur eine geringe bzw. überhaupt keine antigene Wirkung aufweisen, die jedoch in der Lage sind primär auf unspezifische Art und Weise die körpereigenen Abwehrmechanismen zu induzieren. Nach heutigen Erkenntnissen ist eine Vielzahl von Stoffen in der Lage, die Immunabwehr zu stimulieren, wobei insbesondere verschiedene Mineralien, zum Beispiel $Al(OH)_3$, $MgSO_4$, Beryllium, pflanzliche Öle mit oder ohne Zusatz von Mykobakterien sowie eine Reihe von pflanzlichen Wirkstoffgruppen wie Alkaloide, Sesqui- und Diterpenverbindungen, Chinone und Polysaccharide zu nennen sind. Der gesamte Komplex der Immunstimulierung wird ausführlich beschrieben von zum Beispiel Chedid, L., et al. "Immunstimulation", Springer-Verlag , Heidelberg/New York, 1980; Heidelberger, M., "Structure and Immunological Specificity of Polysaccharides"; Fortschritte der chem. org. Naturst. 42, 288 (1982); Drews, J., "Immunpharmakologie", Springer Verlag, 1986, G. Dannhardt, Therapeutikon 11, November 1988, 653; "Immunopotention": Ciba Foundation Symposium, Elsevier, Amsterdam 1973, "Immunopharmacology of Infectious Diseases, Ed. J.A. Majde, Alan R. Liss, New York, 1987.

Die genaue Wirkungsweise immunstimulierender Substanzen konnte in den meisten Fällen bis heute nicht endgültig geklärt werden. Generell zeigen diese Substanzen unter anderem einen Einfluß auf die Funktion und/oder Proliferation von immunkompetenten Zellen, wobei sie jedoch keine Gedächtnisreaktion hinterlassen. Dies bedeutet, daß als Wirkungsziele für die immunstimulierenden Substanzen die Makrophagen und Granulocyten sowie T und B-Lymphozyten im Vordergrund stehen. Die Wirkung kann auf direktem oder indirektem Wege, zum Beispiel über das Komplementsystem oder über Lymphozyten, über die Produktion von Kininen, zum Beispiel Interferon, Interleukine, Tumor-Nekrose-Faktor, Colony stimulation factor und andere, sowie über eine Steigerung von Makro- und Mikrophagocytose erfolgen. Wegen der Verflechtung von unspezifischen und spezifischen Abwehrmechanismen ist dabei mit Kaskadeneffekten und der gleichzeitigen Beeinflussung mehrerer Abwehrmechanismen zu rechnen.

In der Medizin ergeben sich als bevorzugte Anwendungsgebiete für die Immunstimulation vor allem die Therapie von Mischinfektionen, chronischpersistierenden, chemotherapieresistenten, bakteriellen und viralen Infektionen, die Prophylaxe von opportunistischen Infektionen bei Risikopatienten, die Therapie der malignen Erkrankungen und in gewissem Umfang auch die Behandlung von Autoimmunerkrankungen. Immunstimulantien können auch bei der Zytostatika-Therapie zur teilweisen Kompensation der damit verbundenen Immunsuppression eingesetzt werden.

Es ist bekannt, daß verschiedene Polysaccharide, insbesondere aus Pilzen und höheren Pflanzen, in der Lage sind die Aktivität des Immunsystems zu steigern. Zu den bekanntesten Immunstimulantien aus dieser Reihe gehören die $\alpha$ -1,3/1,6-Glukane Lentinan und Schizophyllan, vergleiche zum Beispiel "Immunpharmacology of Infectious Diseases", Springer Verlag 1986.

Aus der EP-A 0 246 069 ist ein wäßriger Extrakt aus Pflanzen der Gattung Nerium mit proliferationshemmenden Eigenschaften bekannt.

Aufgabe der vorliegenden Erfindung ist es neue Polysaccharide mit immunstimulierender und antiproliferativer Wirkung, Verfahren zu deren Gewinnung und ein Arzneimittel enthaltend diese Polysaccharide zur Verfügung zustellen.

Überraschenderweise hat die Anmelderin gefunden, daß aus Teilen, insbesondere den Blättern und Stengeln des Nerium oleander Baumes, Poly saccharide mit immunstimulierender und antiproliferativer Aktivität isoliert werden können.

Eines der erfindungsgemäßen Polysaccharide setzt sich im Gegensatz zu den bisher bekannten Lentinan- und Schizophyllan-Polysacchariden praktisch ausschließlich aus $\alpha$ (1→4) verknüpften D-Galakturonsäureeinheiten zusammen, deren Carboxylgruppen zu ca. 90% methyliert sind. Das Molekulargewicht dieses Polysaccharids wurde mit 30 000 bis 40 000 D, bevorzugt mit ca. 35000 D, in der HPLC-Gelpermeationschromatographie ermittelt.

Es wurde weiterhin gefunden, daß ein Polysaccharidgemisch, das als Zwischenprodukt erhalten wird nicht nur eine allgemein immunstimulierende Aktivität, sondern im besonderen auch über eine die Zellproliferation hemmende Aktivität verfügt. Dieses Polysaccharidgemisch, das man durch Ausfällung der Polysaccharide aus dem Extrakt der Pflanze erhält, enthält im wesentlichen das oben beschriebene $\alpha$ (1→4) verknüpfte Galakturonan, sowie weitere Galakturonane unterschiedlichen Molekulargewichts und auch Oligosaccharide.

Es wurde weiterhin ein niedermolekulares Polysaccharidgemisch isoliert, dessen Molekulargewicht mit etwa 2500 bis 12 000 D in der HPLC-Gelpermeationschromatographie ermittelt wurde. Dieses liegt damit deutlich unter dem Molekulargewicht (ca. 20 000 D) der bislang bekannten Polysaccharide mit immunstimulierender Wirkung. Dieses Polysaccharidgemisch, das man aus dem wäßrigen Extrakt der Pflanze Nerium oleander erhält, enthält drei Unterfraktionen unterschiedlichen Molekulargewichts und unterschiedlicher Zusammensetzung.

Die Überprüfung der immunstimulierenden Wirkung der erfindungsgemäßen Polysaccharide erfolgte durch in vitro Verfahren, die die Leistungsfähigkeit des mononukleären Systems, sowie die Stimulierbarkeit von T und B-Lymphozyten zu messen gestatten.

Bei diesen Untersuchungen wurde überraschend gefunden, daß die erfindungsgemäßen Polysaccharide, bzw. Polysaccharidgemische, insbesondere die Sekretion des Tumor-Nekrose-Faktors deutlich zu steigern vermögen. Der Tumor-Nekrose-Faktor wird von den Makrophagen bei Induktion gebildet und in die Blutbahn abgegeben. Er spielt in der Tumorabwehr eine wichtige Rolle.

Auch in weiteren Tests zeigten die erfindungsgemäßen Polysaccharide und deren Gemische immunstimulierende Aktivität.

Als Ausgangsmaterial zur Isolierung der erfindungsgemäßen Polysaccharide sind Stengel und Blätter des Oleanderbaumes (Nerium oleander) geeignet. Vorzugsweise verwendet man die Blätter.

Das Verfahren zur Isolierung der erfindungsgemäßen Polysaccharide besteht im wesentlichen darin, daß man das zerkleinerte Ausgangsmaterial mit der ca. 5-fachen Menge destillierten Wassers, für 2 bis 4, vorzugsweise 2,5 bis 3 Stunden kocht, anschließend die festen Teile abfiltriert, den Extrakt auf Raumtemperatur abkühlen läßt und nochmals filtriert.

Das hochmolekulare Polysaccharid erhält man aus dem Extrakt über eine Polysaccharidrohfraktion als Zwischenprodukt mit immunstimulierender Aktivität durch Fällung mit Alkohol, oder Komplexbildung mit Schwermetallsalzen oder quartären Ammoniumsalzen. Vorzugsweise fällt man die Polysaccharide mit Ethanol, den man im Verhältnis von 3:1 bis 1:3, vorzugsweise im Verhältnis 1:1 zusetzt. Nach Stehenlassen für mindestens 12 Stunden filtriert man ab, nimmt den Rückstand in destilliertem Wasser auf und wiederholt die Fällung zweimal. Den letzten Rückstand, den man wiederum in Wasser aufnimmt kann man gegebenenfalls lyophilisieren.

Die Rohfraktion trennt man durch bekannte Gelchromatographieverfahren, zum Beispiel unter Verwendung einer Biogel® P-60 Säule, in nieder- und hochmolekulare Bestandteile auf.

Aus der hochmolekularen Fraktion isoliert man anschließend durch Ionenaustauschchromatographie, zum Beispiel unter Verwendung einer DEAE-Sepharose® C 6-B Säule, durch Elution mit einem Salzgradienten, zum Beispiel einem 0-1m NaCl Gradienten, das erfindungsgemäße Polysaccharid bei einer NaCl Konzentration von ca. 0,2 m.

Das Verfahren zur Isolierung der erfindungsgemäßen niedermolekularen Polysaccharide bzw. des Gemisches besteht im wesentlichen darin, daß man das Filtrat nach dem Extraktionsschritt wie vorstehend beschrieben, lyophilisiert, das gefriergetrocknete Material in $H_2O$ dest. aufnimmt und gegen $H_2O$ dest. dialysiert (Ausschlußgrenze ca. 10 000 D). Das gefriergetrocknete Dialysat behandelt man dann mit Alkohol und nach Zentrifugation trennt man den Niederschlag mit bekannten Verfahren der Gelchromatographie weiter auf.

## Beispiel 1

Isolierung und Charakterisierung des hochmolekularen Polysaccharids aus Nerium oleander.

Die Ausbeute an Polysaccharid variiert nach Herkunft und Erntezeit des Ausgangsmaterials.

Alle im folgenden beschriebenen Arbeiten wurden, wenn nicht anders angegeben, bei 4 °C ausgeführt.

Die Blätter des Nerium oleander Baumes wurden zerkleinert und in der ca. 5-fachen Menge destillierten Wassers 3 Stunden lang gekocht. Die festen Anteile wurden abfiltriert und nach Abkühlen auf Raumtemperatur wurde ein weiteres mal filtriert. Das Filtrat wurde mit Ethanol (96%) 1:1 versetzt. Diese Lösung wurde für 12 Stunden stehen gelassen, die gebildete Gelsuspension abfiltriert und der Rückstand in destilliertem Wasser aufgenommen. Diese Lösung wurde wieder mit Ethanol (96%) 1:1 versetzt und für 12 Stunden stehengelassen. Die gebildete Gelsuspension wurde abfiltriert, der Rückstand in destilliertem Wasser gelöst und ein drittes mal mit Ethanol (96%) 1:1 versetzt. Nach Stehenlassen für mindestens 12 Stunden wurde die Gelsuspension abfiltriert, der Rückstand in destilliertem Wasser aufgenommen und lyophilisiert. Auf diese Weise wurde ein Rohextrakt als Zwischenprodukt erhalten.

Zur Gewinnung des erfindungsgemäßen Polysaccharids wurden die niedermolekularen Substanzen von den hochmolekularen Substanzen schonend getrennt. Dazu wurde der Rohextrakt in Wasser gelöst, die Lösung zentrifugiert und der Überstand auf eine Biogel P-60-Säule gegeben und mit destilliertem Wasser eluiert, wobei zwei Fraktionen erhalten wurden. Die erste Fraktion enthielt ein Polysaccharidgemisch vom Molekulargewichtsbereich 17000 bis 120000 D. Die zweite Fraktion (MG ca. < 10000 D) enthielt niedermolekulare Poly- und Oligosaccharide und andere Substanzen.

Das erfindungsgemäße Polysaccharid wurde durch Ionenaustauschchromatographie aus der hochmolekularen Fraktion, z.B. durch Chromatographie über DEAE Sepharose® CL 6 B, erhalten. Dazu wurde die hochmolekulare Fraktion in Wasser gelöst und auf die mit DEAE Sepharose® gefüllte Säule (30 cm x 2 cm) gegeben und mit einem 0-1 m NaCl Gradienten eluiert. Dabei eluierte das erfindungsgemäße Polysaccharid bei ca. 0,2 m NaCl. Es zeigt eine positive optische Drehung von 38,12° (1mg/ml $H_2O$, Raumtemperatur).

Für das erfindungsgemäße Polysaccharid wurde die folgende Struktur ermittelt:

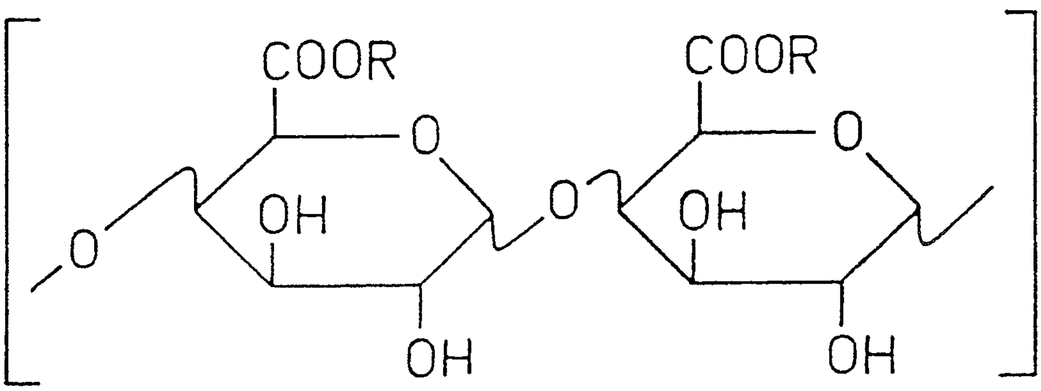

in der der Rest R einen mittleren Methylierungsgrad von etwa 90% aufweist und im übrigen unabhängig voneinander Wasserstoff, Alkali- oder Erdalkalikation bedeutet.

Die Struktur des erfindungsgemäßen Polysaccharids wurde weiterhin durch Methanol-Bestimmung und $^{13}C$-NMR-Spektroskopie untersucht. In diesen Untersuchungen wurde gefunden, daß das Polysaccharid nur aus D-Galakturonsäureeinheiten besteht, die zu ca. 90% methyliert sind.

Die Permethylierungsanalyse lieferte die Bindungsverhältnisse dieses Galakturonans (Hakomori, S.I., J. Biochem. (Tokyo) 55, 205 (1964)).

Die GC-MS Ergebnisse der Permethylierungsanalysen zeigten, daß die Polysaccharidkette aus $\alpha$ (1→4) verknüpften D-Galakturonsäureeinheiten besteht, deren Carboxylgruppen zu ca. 90% methyliert sind. Außer $\alpha$(1→4) glykosidischen Bindungen konnten keine anderen Bindungsverhältnisse nachgewiesen werden. Daraus ergibt sich, daß die Polysaccharidkette linear aufgebaut ist und keine Verzweigungen besitzt.

Zur weiteren Analyse dieses Polysaccharids wurde zunächst das Molekulargewicht bestimmt.

Die Molekulargewichtsbestimmung erfolgt mittels HPLC-Gelpermeationschromatographie (HP-GPC).
Verwendetes HP-GPC-System:
$\mu$-Bondagel E 125 + 5 Bondagel E 500 (Fa. Waters).
Puffersystem: 0,5 m Phosphatpuffer, pH 6

Als Vergleichssubstanzen wurden Dextran T 10, T 40, T 70, T 110, T 2000 und Glucose verwendet.

In dieser Methode wurde für das erfindungsgemäße Polysaccharid ein Molekulargewicht von 30 000 bis 40 000 D, vorzugsweise von ca. 35000 D ermittelt.

Es ist jedoch darauf hinzuweisen, daß das Molekulargewicht in Abhängigkeit der angewandten Untersuchungsmethoden variieren kann.

Durch den Carbazoltest (Bitter, T. und Muir, H.M., Analyt. Biochem. 4, 330 (1962) wurde der Uronsäuregehalt des erfindungsgemäßen Polysaccharids bestimmt.

| | Uronsäuregehalt in % | |
|---|---|---|
| | 1. Test | 2. Test |
| Polysaccharid | 99,9 | 100 |

Zur Bestimmung der Zusammensetzung des erfindungsgemäßen Polysaccharids wurde dieses mit TFA versetzt und 2 Stunden lang bei 121 °C im Trockenschrank erhitzt. Nach der Entfernung von TFA wurde eine Dünnschichtchromatographie, wie anschließend beschrieben, durchgeführt.

Adsorbens: Kieselgel G $F_{254}$-Fertigplatten für die Nano-DC(HPTLC) 20x20 cm (Fa. Merck)
Laufmittelsystem: n-Butanol-Aceton-Essigsäure-Wasser (35:35:10:20)
Detektion: Anilindiphenylaminophosphorsäure

Als Ergebnis wurde gefunden, daß nur D-Galacturonsäure nachweisbar ist.

Die α (1→4) Verknüpfung der D-Galakturonsäure wurde zusätzlich mit Pektinase untersucht. Das Polysaccharid wurde in destilliertem Wasser gelöst und mit Pektinase 3 Stunden bei Raumtemperatur inkubiert. Anschließend wurde mittels Dünnschichtchromatographie die freigesetzte Galakturonsäure nachgewiesen.

Die pharmakologische Wirkung des erfindungsgemäßen Polysaccharids wurde in anerkannten in vitro Tests untersucht.

Da es bislang keine spezifischen Testmethoden zur Überprüfung der immunstimulierenden Wirkung von Substanzen gibt, verwendet man bis heute primär solche in vitro- und in vivo-Verfahren, die den Einfluß von Verbindungen oder Pflanzenextrakten auf den Funktionszustand und die Leistungsfähigkeit des mononuklearen Systems, sowie die Stimulierbarkeit von T- und B-Lymphozyten zu messen gestatten.

Bei diesen Untersuchungen wurde festgestellt, daß das erfindungsgemäße Polysaccharid insbesondere im Tumor-Nekrose Faktor Freisetzungstest wirksam ist.

TNF (Tumor-Nekrose-Faktor) ist ein Protein, das beim Menschen aus 157 Aminosäuren besteht. Es wird bei Induktion in Makrophagen synthetisiert und in die Blutbahn abgegeben. TNF stimuliert das Abwehrsystem und tötet die Tumorzellen ab. Bei dem TNF-Test wird die zu testende Substanz den Versuchstieren injiziert. Danach wird im Blut der Versuchstiere die TNF-Konzentration anhand der nekrotischen Wirkung auf Tumorzellinien gemessen und als Parameter für die stimulatorische Wirkung der Testsubstanzen verwendet.

Der Tumor-Nekrose-Faktor-Test (TNF-T) wurde nach Stimpl, M., Proksch, A., Wagner, H. und Lohmann-Matthes, M.L. Infection and Immunity 46, 845 (1984), durchgeführt. Die Ergebnisse sind in der folgenden Tabelle dargestellt.

| TNF-Test-Ergebnisse: | | |
|---|---|---|
| Polysaccharid | Konzentration (µg) | TNF-Konzentration (U/ml) |
| PS | 50 | 256 |
| PS | 25 | 64 |
| PS | 12,5 | 8 |
| PS | 6,2 | 4 |
| PS = erfindungsgemäßes Polysaccharid | | |

Wie aus der Tabelle entnehmbar ist, bewirkt das erfindungsgemäße Polysaccharid eine deutliche Steigerung der TNF Konzentration, d.h. es induziert in den Makrophagen die Synthese des TNF.

Die immunstimulierende Wirkung der als Zwischenprodukt erhaltenen Rohfraktion wurde ebenfall im Tumor-Nekrose-Faktor Freisetzungstest, s.o., überprüft. Die Ergebnisse zeigt die folgende Tabelle.

| TNF-T-Ergebnisse: | | |
|---|---|---|
| Polysaccharid rohfraktion | Konzentration (µg) | TNF Konzentration (U/ml) |
| PS | 50 | 1000 |
| PS | 25 | 500 |

Die immunstimulierende Wirkung der Polysaccharidrohfraktion wurde zusätzlich im Granulozyten-Test nach Brandt untersucht (Brandt, L., Scand. J. Haemat.(Suppl.)2 (1967).

Im Granulozyten-Test nach Brandt wird durch in vitro Versuche die Zahl der phagozytierten Hefezellen oder Bakterien durch eine Granulozytenfraktion, die aus Humanserum gewonnen wird, unter dem Mikroskop bestimmt. Man mißt die prozentuale Steigerung der Phagozytose durch die erfindungsgemäßen Polysaccharide. Die Ergebnisse sind in der folgenden Tabelle dargestellt.

| Polysaccharid fraktion | Phagozytosewerte in % bei Konz. (mg/ml) | | | | |
|---|---|---|---|---|---|
| | $10^{-1}$ | $10^{-3}$ | $10^{-4}$ | $10^{-5}$ | $10^{-6}$ |
| Rohfraktion (Polysaccharidgemisch) | 95 | 74 | 70 | 42 | 32 |

Wie die Ergebnisse in der Tabelle zeigen, bewirkt das als Zwischenprodukt erhaltene Polysaccharidgemisch eine deutliche Steigerung der Phagozytose, die mit den im TNF-Freisetzungstest erhaltenen Stimulierungsdaten gut korrelieren.

Dieses Zwischenprodukt besitzt weiterhin eine die Zellvermehrung hemmende Wirkung (antiproliferative Wirkung).

Beispiel 2:

Isolierung und Charakterisierung des niedermolekularen Polysaccharidgemisches bzw. der niedermolekularen Polysaccharide aus Nerium oleander Blättern.

Die Ausbeute an Polysaccharid variiert nach Herkunft und Erntezeit des Ausgangsmaterials. Alle im folgenden beschriebenen Arbeiten wurden, wenn nicht anders angegeben, bei 4 °C ausgeführt.

Die Blätter des Nerium oleander Baumes wurden zerkleinert und in der ca. 5-fachen Menge destilliertem Wasser ca. 3 Stunden lang gekocht. Anschließend wurden die festen Anteile abfiltriert und nach Abkühlen auf Raumtemperatur wurde ein weiteres mal filtriert. Das Filtrat wurde lyophilisiert, der Rückstand in destilliertem Wasser gelöst und in einem Dialysierschlauch (Molekulargewichtsausschlußgrenze: ca 10 000 D) gegen destilliertes Wasser 3 x 24 Stunden lang dialysiert. Nach jeweils 24 Stunden wurde das Dialysat gegen destilliertes Wasser ausgewechselt.

Zur Gewinnung des erfindungsgemäßen Polysaccharidgemisches wurde das Dialysat lyophilisiert, der Rückstand mit Methanol versetzt, geschüttelt und 10 Minuten lang bei 3000 UPM zentrifugiert. Dieser Vorgang wurde 3 x wiederholt.

Im weiteren Trennungsgang wurde der nach der Zentrifugation erhaltene Niederschlag in destilliertem Wasser gelöst, auf eine Sephadex®-LH-20 Säule (75 cm x 2 cm) aufgetragen und mit destilliertem Wasser eluiert. Durch die Sephadex®-LH-20-Säule wurde der Niederschlag in mehrere Fraktionen getrennt. Die erste Fraktion (NOAG-II) enthält ein Gemisch von Polysacchariden mit Molekulargewichten zwischen ca. 2 500 und 12 000 D.

Zur Isolierung der Unterfraktion-Polysaccharide aus NOAG-II wurde diese Fraktion, gegebenenfalls nach Lyophylisation, in destilliertem Wasser gelöst, auf eine mit Sephadex®-G-50 gefüllte Säule (95 cm x 1 cm) gegeben und mit destilliertem Wasser eluiert. Durch diese Chromatographie wurde das Gemisch in drei Polysaccharidfraktionen getrennt. Die Unterfraktionen werden im folgenden als NOAG-III, NOAG-IV, NOAG-V bezeichnet.

Durch Zugabe von 15%iger Trichloressigsäure zu den in Wasser gelösten Polysaccharidfraktionen können ca. 5 - 7% Proteinanteile entfernt werden.

Zur Charakterisierung des erfindungsgemäßen Polysaccharidgemisches wurden die folgenden Untersuchungen durchgeführt.

Chemische Untersuchungen:

1. Qualitative Zuckerbestimmung:

Zur Hydrolyse wurden die Fraktionen mit 2 N TFA versetzt und 2 Stunden lang bei 121 °C im

Trockenschrank erhitzt. Nach der Entfernung von TFA wurde eine Dünnschichtchromatographie durchgeführt.
Adsorbens: Kieselgel G $F_{254}$ Fertigplatten für die Nano-DC(HPTLC) 20 x 20 cm (Fa. Merck).
Laufmittelsystem: n-Butanol-Aceton-Essigsäure-Wasser (35:35:10:20).
Detektion: Anilindiphenylaminophosphorsäure.

Als Ergebnis wurde gefunden, daß die folgenden Zucker nachgewiesen werden können:
Galakturonsäure
Arabinose
Rhamnose
Galactose
Xylose
Glucose

2. Quantitative Neutralzuckerbestimmung:

Die Polysaccharidunterfraktionen wurden hydrolisiert, anschließend zu den Alditolacetaten derivatisiert (BLAKENEY A.B. et. al. Carbohydr. Res. 113(1983)291) und die Zucker gaschromatographisch bestimmt.
Gerät: Perkin-Elmer 900
Säule: Glas, 6ftx2mm, GP3% SP-23-30 auf 100/200 Supelcoport.
Temperatur: 210° C

Die molaren Verhältnisse der Neutralzuckerzusammensetzung zeigt die folgende Tabelle.

| Fraktionen | Rhamnose : | Arabinose : | Xylose : | Galactose : | Glucose |
|---|---|---|---|---|---|
| NOAG II | 0,3 | 0,2 | 0,2 | 0,9 | 1,0 |
| NOAG III | 1,1 | 4,4 | --- | 3,2 | 1,0 |
| NOAG IV | 0,4 | 3,9 | --- | 2,0 | 1,0 |
| NOAG V | 0,2 | 0,9 | 0,3 | 0,9 | 1,0 |

Die Berechnung der Molverhältnisse ist auf Glucose gleich 1,0 bezogen.

3. Uronsäurebestimmung:

Mit Hilfe des Carbazoltests von Bitter, T. und Muir, H.M. Analyt. Biochem. 4, 330 (1962) wurde der Uronsäuregehalt der Polysaccharidfraktionen mit 40 bis 70% bestimmt.

4. Molekulargewichtsbestimmung:

Die Molekulargewichte der isolierten Polysaccharide wurden mittels HPLC-Gelpermeationschromatographie (HP-GPC) ermittelt.
HP-GPC-System:
μ-Porasil GPC 60 A + μ-Bondagel E-500.
Puffersystem:
0,05 m Phosphatpuffer pH 6,0 mit 0,15 m NaCl
Vergleichssubstanzen:
Glucose, Maltotriose, Maltoheptaose, Dextransulfat 5 000, Dextran T-10, T-40, T-70, T-110, T-2000.

| Ergebnisse: | |
|---|---|
| Polysaccharid | Molekulargewicht (D) |
| NOAG III<br>NOAG IV<br>NOAG V | 10 000 bis 12 000<br>5 000 bis 6 000<br>2 500 bis 3 500 |

5. Proteingehalt:

Der Proteingehalt der einzelnen Fraktionen wurde nach LOWRY O.H.,J. Biol. Chem. 193 (1951) 265, bestimmt.

In dieser Untersuchung zeigte sich, daß das Polysaccharidgemisch einen Proteingehalt von ca. 6 bis 10% aufweist.

Optische Drehung:

Die optische Drehung der Polysaccharide wurde mit einem Polarimeter (Perkin-Elmer Polarimeter 241) bei 20 °C gemessen.
Wellenlänge: = 589 nm (Na)
Konzentration c: 0,1 %
Lösungsmittel: dest. Wasser

Es zeigte sich, daß die Polysaccharide stark rechtsdrehend sind. Es wurden Werte zwischen 120° und 150° gefunden.

Pharmakologische Untersuchungen:

Die pharmakologische Wirkung des erfindungsgemäßen Polysaccharidgemisches wurde mit folgenden in vitro Tests untersucht, wobei gefunden wurde, daß das erfindungsgemäße Polysaccharidgemisch insbesondere im Tumor-Nekrose Faktor Freisetzungstest wirksam ist.

Lymphozyten-Transformationstest:

Bei dem Lymphozyten-Transformationstest wird zu den Lymphozytenkulturen mit $^3H$ markiertes Thymidin (ein Baustein der DNS) zugesetzt. $^3H$-Thymidin wird von den proliferierenden Lymphozyten aufgenommen und in die DNS eingebaut. Die in die DNS eingebaute $^3H$-Thymidinmenge ist der Lymphozytenproliferation proportional und kann als Parameter zur Beurteilung der stimulierenden Wirkung von Polysacchariden herangezogen werden.

Als Ergebnis zeigt die folgende Tabelle Mittelwerte aus drei unabhängigen Messungen aus der eine sehr gute Stimulierung der Lymphozytenproliferation durch das Polysaccharidgemisch NOAG II entnehmbar ist.

| Fraktion | Lymphozytenstimulation (%) bei Substanzkonzentration (mg/ml) | | | | |
|---|---|---|---|---|---|
| | $10^{-1}$ | $10^{-2}$ | $10^{-3}$ | $10^{-4}$ | $10^{-5}$ |
| NOAG II | 89 | 55 | 38 | 29 | 54 |

Tumor-Nekrose-Faktor-Test (TNF):

Durchführung wie in Beispiel 1 beschrieben.

Als Ergebnis wurde gefunden, daß NOAG II die Makrophagen sehr gut zur TNF-Bildung stimuliert, wobei 1,5 µg NOAG II/ml Medium über 500 U ml TNF induzieren.

Die immunstimulierende Wirkung das Polysaccharidgemisch NOAG II wurde zusätzlich im Granulozytentest nach Brandt (Brandt, L., Scand. I. Haemat. (Suppl.) 2, 1967), wie in Beispiel 1 beschrieben, untersucht.

Das Ergebnis zeigt die folgende Tabelle (Mittelwerte aus zwei unabhängigen Messungen).

| Fraktion | Phagozytosewerte in % bei Konzentration (mg/ml) | | | | | |
|---|---|---|---|---|---|---|
| | $10^{-1}$ | $10^{-2}$ | $10^{-3}$ | $10^{-4}$ | $10^{-5}$ | $10^{-6}$ |
| NOAG II | 70 | 40 | 130 | 21 | 99 | 18 |

Wie die Ergebnisse in der Tabelle zeigen, bewirkt das Polysaccharidgemisch NOAG II eine deutliche Steigerung der Phagozytosewerte, die mit den im TNF-Freisetzungstest erhaltenen Stimulierungsdaten gut korrelieren.

Chemolumineszenz-Test (CL-T):

Bei diesem Test wird die Steigerung der Produktion reaktiver Sauerstoffverbindungen bei Makrophagen und Granulozyten durch die Testsubstanzen bestimmt. Dabei werden die reaktiven Sauerstoffverbindungen mit Verstärkern wie Lucigenin in Reaktion gebracht, die dann zu einer Chemolumineszenz führt. Durch die Messung dieser Chemolumineszenz werden die stimulatorischen Wirkungen von Testsubstanzen auf das Immunsystem ermittelt. Das Ergebnis zeigt die folgende Tabelle (Mittelwerte aus zwei unabhängigen Messungen).

| Fraktion | Chemolumineszenzsteigerung in % bei Konz. (mg/ml) | | | | | |
|---|---|---|---|---|---|---|
| | $10^{-1}$ | $10^{-2}$ | $10^{-3}$ | $10^{-4}$ | $10^{-5}$ | $10^{-6}$ |
| NOAG II | 7,2 | 9,3 | 1,2 | 24,4 | - | 7,6 |

Wie aus der Tabelle ersichtlich, zeigt das Polysaccharidgemisch NOAG II auch in diesem Test Aktivität.

Von den Unterfraktionen zeigte insbesondere NOAG III im TNF Test eine hohe Aktivität.

Die erfindungsgemäßen Polysaccharide bzw. das jeweilige Polysaccharidgemisch können entweder getrennt, oder in Mischungen als Reinsubstanz oder in Form von pharmazeutischen Zubereitungen verabreicht werden, wenngleich es im allgemeinen wirksamer ist, die Verbindung in Form einer pharmazeutischen Zubereitung zu verabreichen. Vorzugsweise liegt die Zubereitung in Form einer Formulierung vor, die

(1) mindestens ein erfindungsgemäßes Polysaccharid bzw. ein Polysaccharidgemisch und

(2) eines oder mehrere geeignete Bindemittel, Trägermaterialien und/oder weitere Hilfsstoffe und

(3) gegebenenfalls weitere therapeutische Wirkstoffe oder Adjuvantien enthält.

Die Trägermaterialien, Bindemittel und/oder Hilfsstoffe müssen pharmazeutisch und pharmakologisch verträglich sein, so daß sie mit den anderen Bestandteilen der Zubereitung vereinbar sind und keine nachteilige Wirkung auf den behandelten Organismus ausüben.

Die Formulierungen schließen jene ein, die für die parenterale (einschließlich subkutane, intradermale, intramuskuläre und intravenöse) oder orale Verabreichung geeignet sind, wenngleich der am besten geeignete Verabreichungsweg von dem Zustand des Patienten abhängt.

Die Herstellung der Formulierungen erfolgt unter Anwendung von an sich auf dem Gebiet der Pharmazie bekannten Verfahren. Alle Verfahren schließen den Schritt ein, ein erfindungsgemäßes Polysaccharid bzw. ein Polysaccharidgemisch (d.h. den Wirkstoff) mit dem Trägermaterial, Bindemittel und/oder Hilfsstoffe, wobei letztere einen zusätzlichen Bestandteil darstellen, zu vermischen bzw. zu vereinigen. Im

allgemeinen werden die Formulierungen dadurch hergestellt, daß man den Wirkstoff mit den flüssigen Trägermaterialien oder den feinteiligen Trägermaterialien oder beiden innig vermischt und dann erforderlichenfalls das erhaltene Produkt in die gewünschte Verabreichungsform bringt. Die erfindungsgemäßen Formulierungen, die für die orale Verabreichung geeignet sind, können in Form von diskreten Einheiten, wie Kapseln, Cachets oder Tabletten, die jeweils eine vorbestimmte Menge des erfindungsgemäßen Wirkstoffes enthalten, sowie in Form von Pulvern oder Granulaten, in Form einer Lösung oder einer Suspension in einer wässrigen oder nicht wässrigen Flüssigkeit, oder in Form einer Emulsion zum Beispiel in Form von Liposomen, vorliegen.

Der Wirkstoff kann auch in Form eines Bolus oder einer Paste vorliegen.

Die Tabletten kann man durch Pressen oder Vergießen herstellen, wobei man gegebenenfalls eines oder mehrere der üblichen Hilfsmittel zugibt.

Die für die parenterale Verabreichung geeigneten Formulierungen schließen sterile Injektionslösungen in wäßrigen oder nicht wässrigen Medien, die Antioxidantien, Puffer, Bakteriostatika und gelöste Materialien, die die Formulierung im Hinblich auf den menschlichen Organismus isotonisch machen, ein. Weiter kann das erfindungsgemäße Polysaccharid bzw. Polysaccharidgemisch in Form von sterilen Suspensionen in wässrigen oder nicht wässrigen Medien, die Suspensionsmittel und Verdickungsmittel enthalten können, vorliegen. Die Formulierungen können als Einzel- oder Mehrfach-Dosis vorliegen, beispielsweise in Form von Ampullen oder dicht verschlossenen Fläschchen und können auch in lyophilisierter Form gelagert werden, so daß es lediglich erforderlich ist, bei notwendiger Verabreichung steriles flüssiges Trägermaterial, beispielsweise für Injektionszwecke geeignetes Wasser, unmittelbar vor Verwendung zuzugeben. Die unmittelbar vor der Verabreichung bereiteten Injektionslösungen und Suspensionen können aus sterilen Pulver, Granulaten und Tabletten der oben beschriebenen Art bereitet werden.

Die erfindungsgemäßen Zubereitungen können neben den vorhin genannten Bestandteilen andere für die in Rede stehenden Formulierungen geeignete Bestandteile enthalten. So können beispielsweise die auf oralem Weg zu verabreichenden pharmazeutischen Zubereitungen Aromastoffe enthalten.

Die zur Applikation geeigneten Mengen des Wirkstoffes variieren in Abhängigkeit vom jeweiligen Therapiegebiet. Im allgemeinen enthält eine Einzeldosis 5 bis 95% aktiven Bestandteil. Dies entspricht bei einer einmaligen Applikation zum Beispiel 50 µg bis 100 mg pro Dosis/Mensch parenteral und 1 mg bis 500 mg peroral. Diese Dosierung kann jedoch in Abhängikeit des Verabreichungsweges, des Patientenzustandes und des Therapiegebietes in breiten Grenzen variieren.

Im folgenden werden beispielhaft einige Formulierungen für die erfindungsgemäßen Zubereitungen beschrieben.

| Tabletten-Formulierung | |
|---|---|
| Formulierung A (Tablette) | mg/Tablette |
| (a) Aktiver Bestandteil | 250 |
| (b) Lactose | 210 |
| (c) PVP | 15 |
| (d) Natriumstärkeglycolat | 20 |
| (e) Magnesiumstearat | 5 |
| | 500 |

| Formulierung B (Kapsel) | mg/Kapsel |
|---|---|
| Aktiver Bestandteil | 250 |
| Vorgelatinisierte Stärke | 150 |
| | 400 |

| Formulierung C | mg/Kapsel |
|---|---|
| Aktiver Bestandteil | 250 |
| Lactose | 150 |
| Mikrokristalline Cellulose | 100 |
| | 500 |

| Formulierung D | mg/Kapsel |
|---|---|
| (a) Aktiver Bestandteil | 250 |
| (b) Lactose | 143 |
| (c) Natriumstärkeglycolat | 25 |
| (d) Magnesiumstearat | 2 |
| | 420 |

| Formulierung E | mg/Kapsel |
|---|---|
| (a) Aktiver Bestandteil | 250 |
| (b) Polyethylenglykol | 350 |
| | 600 |

| Formulierung F (Injektionslösung) | |
|---|---|
| Aktiver Bestandteil | 0,200 g |
| Chlorwasserstoffsäure-Lösung, | 0,1 mol/l q.s. bis ph 4,0 bis 7,0 |
| Natriumhydrxid-Lösung, | 0,1 mol/l q.s. bis ph 4,0 bis 7,0 |
| Steriles Wasser | q.s. bis 10 ml |

| Formulierung G | |
|---|---|
| Aktiver Bestandteil | 0,125 g |
| Steriler, pyrogenfreier Phosphatpuffer vom pH 7, | q.s. bis 25 ml |

| Formulierung H | |
|---|---|
| Aktiver Bestandteil | 0,20 g |
| Benzylalkohol | 0,10 g |
| Tetrahydrofurfuryl-Polyethylan-Glykolether | 1,45 g |
| Wasser für Injektion | q.s. bis 3,00 ml |

| Formulierung I: | |
|---|---|
| Aktiver Bestandteil | 0,2500 g |
| Sorbitol-Lösung | 1,5000 g |
| Glycerin | 2,0000 g |
| Natriumbenzoat | 0,0050 g |
| Geschmacksstoff | 0,0125 ml |
| Gereinigtes Wasser | q.s. bis 5,0000 ml |

**Ansprüche**

1. Polysaccharid der allgemeinen Formel:

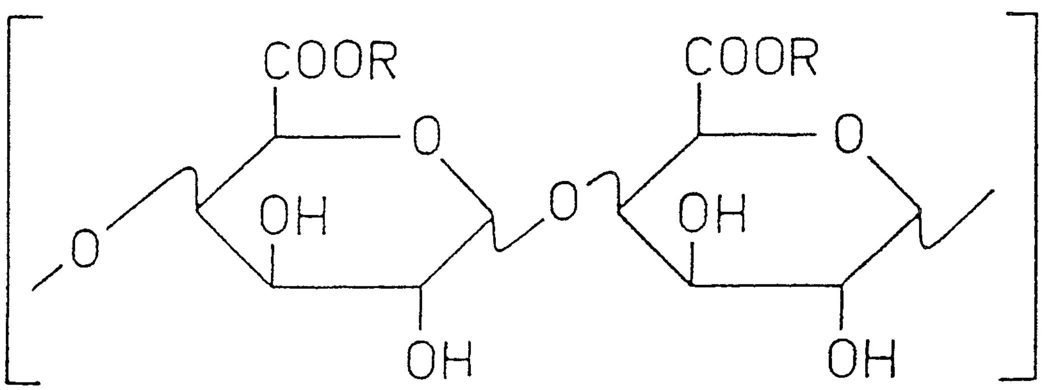

in der der Rest R einen mittleren Methylierungsgrad von etwa 90% aufweist und im übrigen unabhängig voneinander Wasserstoff, Alkali- oder Erdalkalikationen bedeutet und das ein Molekulargewicht von 30 000 bis 40 000 D, bestimmt in der HPLC-Gelpermeationschromatographie, aufweist,

2. Polysaccharid mit einem Molekulargewicht von ca. 10 000 bis 12 000 D, bestimmt in der HP-GPC, enthaltend die Zucker Rhamnose, Arabinose, Galactose und Glucose in einem molaren Verhältnis von 1,1 : 4,4 : 3,2 : 1,0, bezogen auf Glucose = 1,0.

3. Polysaccharid mit einem Molekulargewicht von ca. 5 000 bis 6 000 D, bestimmt in der HP-GPC, enthaltend die Zucker Rhamnose, Arabinose, Galactose und Glucose in einem molaren Verhältnis von 0,4 : 3,9 : 2,0 : 1,0, bezogen auf Glucose = 1,0.

4. Polysaccharid mit einem Molekulargewicht von ca. 2 500 bis 3 500 D, bestimmt in der HP-GPC, enthaltend die Zucker Rhamnose, Arabinose, Xylose, Galactose, Glucose in einem molaren Verhältnis von 2,0 : 0,9 : 0,3 : 0,9 : 1,0, bezogen auf Glucose = 1,0.

5. Verfahren zur Isolierung eines Polysaccharids nach einem der Ansprüche 1 bis 4, dadurch gekennzeichnet, daß man:

a) Teile von Nerium oleander in Wasser für 2 bis 4, vorzugsweise 2,5 bis 3, Stunden kocht,

b) die festen Anteile abtrennt, den Extrakt auf Raumtemperatur abkühlt, filtriert, das Filtrat gegen Wasser mit einer Molekulargewichtsausschließungsgrenze von ca. 10 000 D dialysiert,

c) das Dialysat lyophilisiert, das lyophilisierte Material mit Alkohol behandelt und die unlöslichen Bestandteile abtrennt,

d) aus dem Rückstand mittels Gelchromatographie die Polysaccharide mit einem Molekulargewicht von ca. 3 500 bis 12 000 D isoliert.

6. Verfahren zur Isolierung eines Polysaccharids nach Anspruch 5, dadurch gekennzeichnet, daß man in Stufe b) aus dem Filtrat die Polysaccharide ausfällt, den Niederschlag abfiltriert, in einem Lösungsmittel aufnimmt, dies Lösung gegebenenfalls lyophilisiert, dann durch Gelfiltration eine hoch- und niedermolekulare Fraktion gewinnt und aus der hochmolekularen Fraktion durch Ionenaustauschchromatographie und Elution mit einem Salzgradienten ein saures Polysaccharid isoliert.

7. Verfahren nach Anspruch 5 oder 6, bei dem man die festen Teile in etwa der 5-fachen Menge destilliertem Wasser kocht.

8. Verfahren nach Anspruch 6, bei dem man die Polysaccharide in Stufe b) durch Zusatz von Ethanol (96%) im Verhältnis 1:1 ausfällt.

9. Verfahren nach Anspruch 8, bei dem man die Ethanolfällung zweimal wiederholt.

10. Verfahren nach einem der Ansprüche 6 bis 9, bei dem man das saure Polysaccharid durch Elution mit einem 0-1 m NaCl Gradienten isoliert.

11. Pharmazeutische Zubereitung, enthaltend mindestens ein Polysaccharid nach einem der Ansprüche 1 bis 4 oder ein Polysaccharid erhalten nach einem Verfahren nach einem der Ansprüche 5 bis 10 in Verbindung mit pharmazeutisch annehmbaren Trägerund/oder Hilfsstoffen.